(19) Europäisches Patentamt
European Patent Office
Office européen des brevets

(11) Numéro de publication: **0 005 388**
**B1**

(12) # FASCICULE DE BREVET EUROPEEN

(45) Date de publication du fascicule de brevet: **23.09.81**

(21) Numéro de dépôt: **79400234.5**

(22) Date de dépôt: **11.04.79**

(51) Int. Cl.$^3$: **B 01 J 23/50,**
C 07 D 301/10,
C 07 D 303/04

(54) **Catalyseurs à base d'argent pour la production d'oxyde d'éthylène.**

(30) Priorité: **28.04.78 FR 7812628**
**27.07.78 FR 7822214**

(43) Date de publication de la demande:
**14.11.79 Bulletin 79/23**

(45) Mention de la délivrance du brevet:
**23.09.81 Bulletin 81/38**

(84) Etats Contractants Désignés:
**BE DE FR GB IT NL**

(56) Documents cités:
**CHEMICAL ABSTRACTS, volume 87, n° 12, 19-9-1977, page 449, colonne de gauche, résumé n° 91551r**
**COLUMBUS OHIO (USA)**

(73) Titulaire: **P C U K PRODUITS CHIMIQUES UGINE KUHLMANN**
**Service Propriété Industrielle Tour Manhattan**
**F-92087 PARIS LA DEFENSE 2 Cédex 21 (FR)**

(72) Inventeur: **Cognion, Jean-Marie**
**85, Route de Charly**
**F-69230 St Genis Laval (FR)**
Inventeur: **Kervennal, Jacques**
**134, rue Docteur Locard**
**F-69005 Lyon (FR)**

(74) Mandataire: **Monceaux, Pierre et al,**
**PRODUITS CHIMIQUES UGINE KUHLMANN**
**Service Propriété Industrielle**
**Tour Manhattan Cédex 21 F-92087 Paris La Defense (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

# Catalyseurs à base d'argent pour la production d'oxyde d'éthylène

La présente invention concerne des catalyseurs à base d'argent employés pour la production en phase vapeur de l'oxyde d'éthylène à partir d'éthylène et d'oxygène moléculaire.

De façon générale, la production d'oxyde d'éthylène est effectuée en phase vapeur, dans des réacteurs tubulaires à lit fixe, par réaction de l'oxygène et de l'éthylène sur des phases catalytiques à base d'argent déposées avec les éventuels promoteurs, sur des supports réfractaires et inertes formés principalement d'alumine, de silice-alumine, de magnésie, de pierre ponce, de zircone, d'argile, de céramique, de graphite naturel ou artificiel, d'amiante, de zéolithes naturelles ou artificielles ou de carbure de silicium. L'art antérieur préfère généralement des solides de faibles surfaces spécifiques, inférieures à 10 m2/g, et de volume de porosité élevée pouvant atteindre 60 % en volume. Ces surfaces spécifiques sont déterminées, dans la majorité des cas, par la méthode d'adsorption de l'azote dite méthode B.E.T. décrite par BRUNAUER, EMMET et TELLER dans: "The Journal of American Chemical Society", vol. 60, page 309, 1938, tandis que les mesures de porosité sont effectuées par la méthode dite du porosimètre à mercure préconisée par E.W. WASHBURN dans les "Proceedings of the National Academy of Sciences of USA", vol. 7, page 115, 1927 et décrite par L.C. DRAKE dans "Industrial and Engineering Chemistry", vol. 41, page 780, 1949 et dans "Industrial and Engineering Chemistry-Analytical Edition", vol. 17, page 782, 1945.

Le choix d'un support de texture convenable, conforme aux caractéristiques décrites ci-dessus ne suffit pas car l'activité, la sélectivité en oxyde d'éthylène et la tenue dans le temps des catalyseurs dépendent également de la nature du précurseur de la phase active employée et de la technique utilisée pour le déposer sur les supports.

Il est connu que les catalyseurs à l'argent employés dans l'époxydation de l'éthylène peuvent être préparés à partir de composés métalliques par deux techniques différentes:

a) l'enrobage du support par le compsé métallique en suspension dans un liquide.

b) l'imprégnation du support par le composé métallique en solution dans un solvant judicieusement choisi.

Dans les deux cas les composés d'argent doivent être facilement décomposables et libérer aisément l'argent sur le support. La nature de l'association du composé métallique et du solvant que l'on emploie est très importante, elle peut, combinée avec la texture du support et le traitement de passage au métal, avoir une influence considérable sur l'activité et la sélectivité du catalyseur. Pour obtenir des grains ou des agglomérats de grains d'argent de forme et de taille favorables à l'activité et à la sélectivité en oxyde d'éthylène, on imprègne les supports par des solutions de composés organométalliques de l'argent dont le, ou les substrats organiques comportent un groupement fonctionnel à caractère acide, capable de former un sel d'argent et un ou plusieurs atomes d'azote ou d'oxygène susceptible de former une liaison de coordination avec l'atome métallique.

La premier type de ces composés employés est constitué par des complexes intramoléculaires de l'argent dans lesquels le groupement acide et les groupements fonctionnels, susceptibles de complexer le métal font partie de la même molécule. On peut citer comme exemples l'imprégnation des supports par une solution aqueuse de lactate d'argent revendiquée dans le brevet français 1.295.395 du 23.3.61 ou par une solution pyridinique des dérivés de l'argent des acides anthranilique, picolinique ou salicylique revendiquée dans la demande de brevet français 77.33866 du 10.11.77 de la demanderesse. La technique décrite dans le brevet français 2.023.986 du 21.11.69 est différente mais on peut supposer qu'elle consiste à former, en milieu aqueux, sur les supports les dérivés de l'argent des acides tartrique, citrique, malique ou isomalique.

Le deuxième type de ces composés employés est constitué par l'association d'un sel minéral ou organique de l'argent et d'une molécule possédant un ou plusieurs groupements complexants susceptibles de créer une liaison de coordination avec le métal. On peut citer les brevets français 2.117.183 et 2.271.869 des 30.11.71 et 16.5.75 qui revendiquent l'impregnation à l'aide de solutions aqueuses, éventuellement ammoniacales, d'oxalate ou de carbonate d'argent associé à des composés comme l'éthylène diamine et l'éthanolamine. Un exemple de préparation est décrit à partir du composé

$$Ag_2\,(CH_2NH_2 — CH_2\,NH_2)_2\,C_2O_4$$

— le brevet français 2.253.747 du 4.12.78 qui semble préférer l'ammoniac comme agent complexant des carboxylates d'argent mais qui insiste sur l'importance de l'addition à la solution aqueuse d'imprégnation d'une amine réductrice.

— le brevet français 2.351.700 du 18.5.77 qui emploie des solutions aqueuses de complexe de la forme $Ag(amine)_2NO_3$ avec une préférence pour les amines de formule générale:

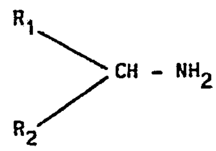

— le brevet français 2.336.974 du 23.11.76 qui cherche à mettre en évidence pour la préparation de catalyseurs à partir de carboxylates d'argent des complexants aminés particuliers pouvant également remplir le rôle de solvant. Il s'agit de polyamines aliphatiques et d'aminoéthers particuliers comme la diéthylènetriamine ou l'oxyde de bis(amino-2 éthyle).

— le brevet français 2.215.267 du 24.1.74 qui emploie comme complexant et solvant de l'acétate d'argent, des composés pyridiniques.

L'importance des caractéristiques des cristallites d'argent ainsi obtenus et notamment leurs tailles, leurs formes et leurs répartitions sur le support a été nettement mise en évidence dans les brevets français 2.271.869 du 16.5.75, 2.148.028 du 26.7.72, 2.352.810 du 25.7.77 et le brevet belge 852.123 du 4.3.77, dans les articles de Peter HARRIOTT et collaborateurs dans le "Journal of Catalysis" vol. 39 (3) page 395 (1975), vol. 21 (1) page 56 (1971) et dans les thèses de J.Wu (1973): "The effect of cristallite size on the activity and selectivity of silver catalysts" à l'Université CORNELL (U.S.A.) et de M.M. JARJOUI (1976): "Etude de l'oxydation partielle de l'éthylène au contact des catalyseurs supportés à base d'argent" à l'Université de LYON (France).

La demanderesse a trouvé que le traitement thermique en atmosphère inerte, réductrice ou oxydante de supports imprégnés par des complexes de sels d'argent avec des imino-éthers cycliques du type oxazole (I), oxazoline-2 (II) et oxazoline-3 (III):

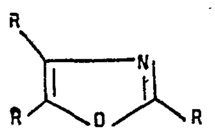

(I)

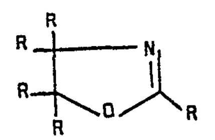

(II)

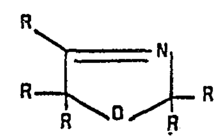

(III)

ou des alkyl oxyimines cycliques du type isoxazole (IV) et isooxazoline-2 (V):

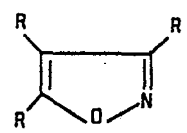

(IV)

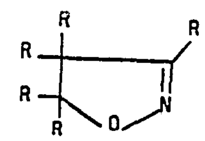

(V)

conduisait à des catalyseurs très actifs dont la sélectivité élevée en oxyde d'éthylène était peu influencée par la teneur en $CO_2$ dans les réactifs et par l'augmentation de productivité. Dans les formules générales de ces composés (I à V) R représente un atome d'hydrogène, un groupement alkyle ayant de 1 à 10 atomes de carbone, ou un groupement phényle. Les observations au microscope électronique à balayage sur les catalyseurs objets de l'invention ont mis en évidence une distribution homogène et régulière des grains d'argent, d'aspect semi-sphérique et de diamètre équivalent compris entre 0,1 et 1 micron.

Les catalyseurs, objet de la présente invention, ont été préférentiellement préparés par un procédé en deux étapes:

a) imprégnation du support avec une solution du composé d'argent choisi. Cette imprégnation pouvant être effectuée soit par immersion du support dans la solution suivie d'un égouttage ou d'une distillation du solvant, soit par arrosage continu du support dans des conditions de pression et de température permettant l'élimination immédiate du solvant.

b) traitement thermique du produit obtenu de manière à libérer l'argent du composé déposé sur le support.

Les sels d'argent que l'on peut utiliser sont nombreux: nitrate, sulfate, carbonate, formiate, lactate, citrate, salicylate, maléate, malate, malonate, phtalate, tartrate, picolinate anthranilate, complexe du salicylaldéhyde, mais l'on préfère nettement les carboxylates d'argent coprenant 1 à 10 carbones et 1 à 2 fonctions acides par molécule tels que l'acétate, le propionate, le benzoate, l'oxalate, le pivalate.

Ces sels peuvent être remplacés par l'introduction dans la solution d'imprégnation d'argent sous forme d'oxyde et d'une quantité d'acide égale ou de préférence légèrement supérieure à la quantité théorique nécessaire pour la réaction de formation du sel métallique correspondant. On utilise en général un excès molaire de 5 à 25 % d'acide par rapport à la quantité stoechiométrique.

Comme solvant, on emploie avantageusement les nouveaux complexants décrits et objets de la

présente invention, mais la demanderesse a également trouvé que ces hétérocycles constituent d'excellents solubilisants des dérivés de l'argent dans un très grand nombre de solvants ou de mélanges de solvants. Les quantités minimales d'imino-éthers cycliques ou d'alkyloxyimines cycliques à employer étant alors de deux moles d'hétérocycles par mole d'argent, on peut réaliser l'imprégnation avec des solutions concentrées en argent, pouvant atteindre 300 à 400 g par litre, et préparées avec des solvants usuels peu coûteux comme l'eau, éventuellement rendue ammoniacale, les nitriles et principalement l'acétonitrile, l'acylonitrile, le propionitrile, les cétones comme l'acétone et la méthyléthyl cétone, les alcools légers, l'éthylène gylcol et le diéthylène glycol et les amines.

Toutes les techniques qui permettent l'élimination du substrat organique des composés métalliques peuvent être appliquées aux supports imprégnés. Un traitement particulièrement approprié consiste en une décomposition thermique dans les conditions suivantes:

montée de 20 à 280°C à raison de 20°/heure, suivie d'une palier de 10 à 50 heures à 280—300°C. L'opération étant effectuée sous balayage d'azote avec addition possible au cours du traitement de composants oxydants ou réducteurs comme par exemple l'air, l'oxygène, l'hydrogène, le formol ...

Les catalyseurs contiennent de 5 à 25 % d'argent et de façon préférentielle de 8 à 20 %.

La nature et la texture du support sont importantes. On emploie principalement des supports réfractaires comme l'alumine-$\alpha$, les silice-alumines, le carbure de silicium, la zircone. Ces supports doivent posséder une granulométrie élevée, compatible avec un emploi dans les réacteurs industriels en lit fixe. Ils se présentent sous forme d'anneaux, de pastilles, de cassons, de sphères ou de formes extrudées, de dimensions comprises entre 1/8 et 1/2 pouce (3,2 à 13 mm) et de propriétés mécaniques satisfaisantes, ne permettant en aucun cas la formation de poussières au cours de la préparation du catalyseur, de ses manipulations ou de son fonctionnement. Les surfaces spécifiques de ces produits sont inférieures à 10 m2/g mais l'on préfère des surfaces encore plus faibles, inférieures à 2 m2/g et plus particulièrement comprises entre 0,1 et 1 m2/g.

Les porosités apparentes principalement formées de pores de rayons supérieurs à 0,25 microns, doivent être au minimum de 40 à 50% en volume.

On a principalement utilisé pour ces essais un support silice alumine commercialisé par la firme NORTON, de forme sphérique, de granulométrie 1/4 ou 3/16 de pouce (6,5 à 4,9 mm), de surface spécifique 0,30 m2/g et de volume total de porosité de 0,34 cm3/g.

La demanderesse a également vérifié, en accord avec l'art antérieur:

a) que certains composés, comme les dérivés halogénes des hydrocarbures, augmentaient la sélectivité de ses catalyseurs lorsqu'ils étaient ajoutés en faibles quantités aux réactifs. Le dichloro-1,2 éthane à une concentration maximale de 1 ppm dans le volume gazeux total est d'un emploi particulièrement intéressant.

b) qu'il était possible d'ajouter à ces catalyseurs dans les teneurs habituelles de 0 à 2 % en poids, tous les promoteurs solides classiques de l'argent tels que : K, Ca, Cs, Ba, Pt, Ni, Sn, Cd, Sr, Li, Mg, Na, Rb, Au, Cu, Zn, La, Ce, Th, Be, Sb, Bi, Ti, Pd, Ir, Os, Ru, Fe, Al.

ces éléments pouvant se trouver dans les catalyseurs finis sous forme métallique ou sous forme d'oxyde ou de composé.

Les exemples 1 à 12 ci-dessus illustrent de façon non limitative la préparation et l'utilisation des catalyseurs selon l'invention. Les résultats obtenus dans ces exemples sont exprimés en taux de transformation globale de l'éthylène et en sélectivité.

— taux de transformation globale de l'éthylène (T.T.G.):

$$\text{T.T.G.} = \frac{\text{Nombre de moles d'éthylène transformées}}{\text{Nombre de moles d'éthylène introduites}} \times 100$$

— sélectivité de la transformation en oxyde d'éthylène (S.O.E.):

$$\text{S.O.E.} = \frac{\text{Nombre de moles d'oxyde d'éthylène formées}}{\text{Nombre de moles d'éthylène transformées}} \times 100$$

Exemple 1.

On charge dans un ballon à solides, monté sur un évaporateur rotatif, 42,6 g de support NORTON SA 5239 de granulométrie 4/16è de pouce (6,5 mm). On porte la température du bain d'huile de l'évaporateur à 130°C et on laisse dégazer le support pendant une heure sous pression réduite. On introduit ensuite goutte à goutte, sur le support agité, une solution de 16,5 g d'acétate d'argent dans 108 g de méthyl-2 oxazoline-2. Dans ces conditions, l'évaporation du solvant est instantanée. Après introduction de la totalité de la solution, le support imprégné est séche, est transféré dans un réacteur tubulaire pour y subir un traitement thermique afin de décomposer l'acétate et de libérer l'argent métallique. Ce traitement est effectué sous courant d'azote avec une montée de température à

20°/heure jusqu'à 270°C, température à laquelle on reste en palier pendant 18 heures. La teneur en argent dans le catalyseur, obtenue par dosage, est de 17 % en poids. Un examen au microscope électronique à balayage, associé à un dispositif de microanalyse aux rayons X permet de noter une répartition régulière sur le support de cristallites d'argent d'aspect hémisphériques de diamètre équivalent compris entre 0,2 et 0,4 microns.

On place 30 ml de ce catalyseur dans un réacteur constitué par un tube en acier inoxydable de 600 mm de longueur et de 16 mm de diamètre intérieur. Les réactifs, admis par le bas du tube, sont préchauffés sur un lit d'anneaux de porcelaine de 200 mm de hauteur, qui supporte egale--ment la charge du catalyseur. Le chauffage de l'ensemble est assuré par une circulation d'huile dans une double enveloppe. Les gaz entrant et sortant du réacteur sont analysés en ligne à l'aide d'un chromatographe à double détection : un détecteur à ionisation de flamme pour l'oxyde d'éthylène, le méthane, le formol, le propylène, le propane, le méthanol et l'acétaldéhyde et un détecteur à conductibilité thermique pour l'oxygène azote, le dioxyde de carbone l'ethylène et l'eau. Deux colonnes de diamètre 1/8é de pouce et de longueur 2,5 mètres, montées en série, sont remplies l'une de chromosorb 101, l'autre de Porapak Q.

On fait passer à travers le lit de catalyseur disposé dans le réacteur un mélange air-éthylène 50 %—50 % à la pression atmosphérique, pendant 47 heures entre 177°C et 185°C. On introduit ensuite dans le réacteur un courant gazeux de 9 000 l normaux/heure et par litre de catalyseur constitué d'un mélange de 13 % d'éthylène, 5 % d'oxygène, 82 % d'azote et 35 ppb de dichloro-1,2 éthane. A 206°C, pour un taux de transformation globale de l'éthylène de 5 %, la sélectivité en oxyde d'éthylène est de 78,5 %.

Exemple 2

On introduit goutte à goutte sur 42,6 g de support NORTON SA 5239 de granulométrie 4/16é de pouce (6,5 mm), placé dans un ballon à solides, monté sur un évaporateur rotatif et préalablement dégazé à 100° pendant une heure sous pression réduite, une solution de 16,5 g d'acétate d'argent dans 108 g d'oxazole. Le bain d'huile de l'évaporateur étant á 100°C l'oxazole est distillé au fur et à mesure de son introduction. Le support imprégné est ensuite traité thermiquement selon la description faite dans l'exemple 1. Le dosage indique une teneur en argent dans le catalyseur de 16,5 % en poids. Un examen au microscope électronique à balayage, associé à un dispositif de microanalyse aux rayons X montre une répartition régulière sur le support de cristallites d'argent d'aspect hémisphériques de diamètre équivalent compris entre 0,3 et 0,5 microns.

On place 30 ml du catalyseur dans le réacteur décrit dans l'exemple 1. On y fait passer un courant gazeux d'air et d'éthylène 50—50 % pendant 30 heures entre 138°C et 184°C. On introduit ensuite sous 20 bars un mélange gazeux de 9 000 litres normaux par heure et par litre de catalyseur, formé de 13 % d'éthylène, 5 % d'oxygène, 35 ppb de dichloro-1,2 éthane et de différentes teneurs en $CO_2$ et azote.

Les résultats obtenus sont indiqués dans le tableau N° 1 ci-dessous:

| Composition des gaz entrant an % | | | | Température (°C) | T.T.G. $C_2H_4$ (%) | Sélectivité S.O.E. (%) |
|---|---|---|---|---|---|---|
| $C_2H_4$ | $O_2$ | $CO_2$ | azote | | | |
| 13 | 5 | 0 | 82 | 218 | 5 | 79 |
| 13 | 5 | 10 | 72 | 229 | 5 | 77,5 |
| 13 | 5 | 14 | 68 | 225 | 5 | 77 |

Exemple 3

On place dans un ballon à solides, monté sur un évaporateur rotatif, 36 g de support NORTON SA 5239 de granulométrie 3/16è pouce (4,9 mm) qu'on laisse dégazer pendant une heure à 120°C sous pression réduite. Parallèlement on verse 5 g d'acide acétique dans 100 g de méthyl-2 oxazoline-2 et on y dissout 8,5 g d'oxyde d'argent. On introduit ensuite goutte à goutte cette solution sur le support L'analyse, après un traitement thermique effectué d'une façon identique à celui décrit dans l'exemple 1, indique une teneur en argent dans le catalyseur de 13,5 % en poids. On charge 30 ml de celui-ci dans le réacteur décrit dans l'exemple 1. Après un pré-traitement d'activation par un mélange air-éthylène à 50 %—50 % pendant 48 heures entre 182°C et 218°C, effectué à pression atmosphérique, on introduit les réactifs sous 20 bars dans les proportions et conditions suivantes : éthylène 13 %, oxygène 5 %, azote 82 %, dichloro-1,2 éthane 35 ppb, débit gazeaux 9 000 litres normaux par heure et par litre de catalyseur. A 218°C après 58 heures de marche, la sélectivité en oxyde d'éthylène est de 77 % pour un taux de transformation globale de l'éthylène de 5 %.

Exemple 4

On charge dans un ballon à solides, monté sur un évaporateur rotatif, 37,5 g de support NORTON SA 5239 de granulométrie 3/16è pouce (4,9 mm). En opérant de la même façon que dans l'exemple 1, on prépare un catalyseur en imprégnant ce support à l'aide d'une solution préalablement formée de 8,9 g d'oxyde d'argent dans 10,3 g d'acide benzoïque et 120 g de méthyl-2 oxazoline-2. L'analyse après traitement thermique indique une teneur en argent dans le catalyseur de 14,2 % en poids.

On charge 30 ml de ce catalyseur dans le réacteur décrit dans l'exemple 1. Après un pré-traitement d'activation par un mélange air-éthylène à 50 %—50 % pendant 114 heures entre 198° et 213°C, à la pression atmosphérique, on introduit les réactifs sous 20 bars dans les proportions suivantes : éthylène 13 %, oxygène 5 %, azote 82 %, 35 ppb de dichloro-1,2 éthane. Le débit gazeux total est de 9 000 litres normaux par heure et par litre de catalyseur. A 232°C la sélectivité en oxyde d'éthylène est de 76,5 % pour un taux de transformation globale de l'éthyléne de 5 %.

Exemple 5

On place dans un ballon à solides monté sur un évaporateur rotatif, 36,5 g de support NORTON SA 5239 de granulomérie 3/16è pouce (4,9 mm). En opérant suivant le mode opératoire décrit dans l'exemple 1, on prépare un catalyseur en imprégnant ce support à l'aide d'une solution préalablement formée de 8,6 g d'oxyde d'argent, 8,33 g d'acide pivalique et 104 g de méthyl-2 oxazoline-2. L'analyse après traitement thermique indique que la teneur en argent est de 16,6 % en poids dans le catalyseur. On introduit 30 ml de celui-ci dans le réacteur décrit dans l'exemple 1. Après un pré-traitement d'activation, effectué à la pression atmosphérique par un mélange air-éthylène à 50 %—50 % pendant 48 heures entre 184°C et 200°C, on fait passer les réactifs gazeux dans les proportions suivantes : éthylène 13 %, oxygène 5 %, azote 82 %, dichloro-1,2 éthane 35 ppb, le débit total étant de 9 000 litres normaux par heure et par litre de catalyseur. Dans ces conditions, à 224°C, la sélectivité en oxyde d'éthylène est de 77 % pour un taux de transformation globale de l'éthylène de 3,4%.

Exemple 6

On charge 29,5 g de support NORTON SA 5239 de granulométrie 4/16è de pouce (6,5 mm) dans un ballon à solides monté sur un évaporateur rotatif. Suivant le mode opératoire décrit dans l'exemple 1, on prépare un catalyseur en imprégnant le support à l'aide d'une solution de 9 g d'oxyde d'argent dans 5 g d'acide acétique, 28,2 g de méthyl-2 oxazoline-2 et 220 ml d'acetonitrile. Après traitement thermique, l'analyse indique que la teneur en argent du catalyseur est de 19 % en poids. On charge 30 ml de ce catalyseur dans un réacteur constitué par un tube en acier inoxydable de 355 mm de longueur et de 16 mm de diamètre intérieur, chauffé par un bain de nitrates fondus. Les réactifs, admis par le bas du réacteur, sont préchauffés sur un remplissage de porcelaine de 42 mm de hauteur. Le dispositif analytique est indentique à celui décrit dans l'exemple 1.

Après un pré-traitement d'activation par un mélange air-éthylène à 50 %—50 % pendant 30 heures entre 157°C et 175°C à la pression atmosphérique, on introduit les réactifs sous 20 bars dans les proportions suivantes : éthylène 13 %, oxygène 5 %, azote 82 %, dichloro-1,2 éthane 35 ppb. Avec un débit total de 9 000 litres normaux par heure et par litre de catalyseur, on obtient à 195°C une sélectivité en oxyde d'éthylène de 77 % pour un taux de transformation de l'éthylène de 5%.

Exemple 7

Dans un ballon à solides, monté sur un évaporateur rotatif, on charge 36 g de support NORTON SA 5239 de granulométrie 3/16è de pouce. On porte la température du bain d'huile de l'evaporateur à 100°C et on laisse dégazer le support pendant 1 h. 30 sous pression réduite. Parallèlement, dans un erlenmeyer contenant 90 g d'oxazole, on verse 5 g d'acide acétique pur; on y ajoute par petites fractions 8,6 g d'oxyde d'argent. On agite le mélange pendant 1 heure pour faciliter la dissolution puis on laisse reposer pendant 1/2 heure. On introduit ensuite goutte à goutte cette solution sur le support agité de façon à que l'évaporation du solvant soit instantanée. Après introduction de la totalité de la solution, le support, imprégné et séché, est transféré dans un réacteur tubulaire pour y subir un traitement thermique destiné à libérer l'argent métallique. Ce traitement est effectué sous courant d'azote avec une montée de température de 20°/heure jusqu'à 270°C, température à laquelle on reste en palier pendant 18 heures. La teneur en argent du catalyseur, obtenue par dosage, est de 12 % en poids.

On place 30 ml de ce catalyseur dans un réacteur constitué par un tube en acier inoxydable de 600 mm de longueur et de 16 mm de diamètre intérieur. Les réactifs, admis par le bas du tube, sont préchauffés sur un lit d'anneaux de porcelaine de 200 mm de hauteur, qui supporte également la charge du catalyseur. Le chauffage de l'ensemble est assuré par une cirulation d'huile dans une double enveloppe. Les gaz entrant et sortant du réacteur sont analysés en ligne à l'aide d'un chromatographe à double détection : un détecteur à ionisation de flamme pour l'oxyde d'éthylène, le méthane, le formol, le propylène, le propane, le méthanol et l'acétaldéhyde et un détecteur à conductibilité thermique pour l'oxygène, l'azote, le dioxyde de carbone, l'éthylène et l'eau. Deux colonnes de diamètre 1/8 de pouce et de longueur 2,5 mètres, montées en série, sont remplies l'une de chromosorb 101, l'autre de Porapak Q.

On fait passer à travers le lit de catalyseur disposé dans le réacteur un mélange air-éthylène à

50 %—50 %, à la pression atmosphérique, pendant 48 heures entre 156° et 178°C. On introduit ensuite dans le réacteur un courant gazeux de 9 000 litres normaux par heure et par litre de catalyseur, constitué d'un mélange de 13 % d'éthylène, 5 % d'oxygène, 82 % d'azote et 35 ppm de dichloro-1,2 éthane. Après 45 heures sous réactifs, à 217°C, le taux de transformation globale de l'éthylène est de 5 % et la sélectivité en oxyde d'éthylène de 77,5 %.

Exemple 8

On introduit goutte à goutte sur 36,5 g de support NORTON SA 5251 de granulométrie 3/16è de pouce, placé dans un ballon à solides monté sur un évaporateur rotatif et préalablement dégazé à 100°C pendant une heure sous pression réduite, une solution filtrée de 13,5 g de propionate d'argent dans 87 g d'oxazole. En maintenant la température du bain d'huile de l'évaporateur à 100°C, l'oxazole est distillé au fur et à mesure de son introduction. Le support imprégné est ensuite traité thermiquement selon la description faite dans l'exemple 7. La teneur en argent dans le catalyseur est de 12,5 %.

On place 30 ml du catalyseur dans le réacteur décrit dans l'exemple 7. On y fait passer à la pression atmosphérique un courant gazeux d'air et d'éthylène à 50 %—50 % pendant 46 heures entre 140°C et 161°C. On introduit ensuit sous 20 bars un mélange gazeux de 9 000 litres normaux par heure et par litre de catalyseur, formé de 13 % d'éthylène, 5 % d'oxygène, et de 82 % d'azote additionné de 35 ppb de dichloro-1,2 éthane. A 185°C, pour un taux de transformation globale de l'éthylène de 5 %, la sélectivité en oxyde d'éthylène est de 80 %.

Exemple 9

Dans un ballon à solides, monté sur un évaporateur rotatif, on charge 42,5 g de support NORTON SA 5239 de granulométrie 1/4 de pouce. On porte la température du bain d'huile de l'évaporateur à 120°C et laisse dégazer le support pendant 1 heure sous pression réduite. Pendant ce temps on dissout en agitant 16,5 g d'acétate d'argent dans un mélange de 92 g d'eau et de 18 g d'oxazole. On introduit ensuite goutte à goutte la solution sur le support agité. L'analyse, après un traitement thermique identique à celui décrit dans l'exemple 7, indique une teneur en argent dans le catalyseur de 11,5 % en poids. On charge 30 ml de celui-ci dans le réacteur décrit dans l'exemple 7. Après un prétraitement d'activation à l'aide d'une mélange air-éthylène à 50 %—50 % pendant 48 heures entre 162° et 185°C, effectué à pression atmosphérique, on introduit sous 20 bars un débit de 9 000 litres normaux par heure et par litre de catalyseur de réactifs gazeux dans les proportions suivantes : éthylène 13 %, oxygène 5 %, azote 82 % et 35 ppb de dichloro-1,2 éthane. A 228°C après 50 heures de marche, la sélectivité en oxyde d'éthylène est de 77,8 % pour un taux de transformation globale de l'éthylène de 4,5 %.

Exemple 10

On charge dans un ballon à solides, monté sur un évaporateur rotatif 36 g de support CARBORUNDUN SAHT 96 de granulométrie 1/4 de pouce. En opérant de la même façon que dans l'exemple 9, on prépare un catalyseur en imprégnant ce support à l'aide d'une solution préalablement filtrée de 14 g d'acétate d'argent dans 94 g d'oxazole. L'analyse après traitement thermique indique une teneur en argent dans le catalyseur de 16 % en poids.

On charge 30 ml de ce catalyseur dans un réacteur de laboratoire fonctionnant sous pression, constitué par une tube en acier inoxydable de 355 mm de long et de 16 mm de diamètre intérieur, chauffé par un bain de nitrates fondus. Les réactifs admis par le bas du réacteur, sont préchauffés sur un remplissage en porcelaine de 42 mm de hauteur. Les gaz, à l'entrée et à la sortie du réacteur, sont analysés à l'aide d'un dispositif analogue à celui décrit dans l'exemple 7. On fait passer à travers le lit de catalyseur disposé dans le réacteur un mélange air-éthylène 50 %—50 %, à la pression atmosphérique, pendant 48 heures entre 137° et 155°C. On introduit ensuite sous 20 bars, 9 000 litres normaux par heure et par litre de catalyseur d'un mélange gazeux contenant 13 % d'éthylène, 5 % d'oxygène, 82 % d'azote et 35 ppb de dichloro-1,2 éthane. A 191°C, pour un taux de transformation globale de 5 %, la sélectivité en oxyde d'éthylène est de 77 %.

Exemple 11

On introduit dans un ballon à solides monté sur un évaporateur rotatif, contenant 36 g de support NORTON SA 5239 de granulométrie 1/4 de pouce une solution préalablement filtrée de 14 g d'acétate d'argent dans 126 g d'oxazole. On dégaze le support immergé à température ambiante pendant 1 h. 30 sous pression réduite. On chauffe le bain d'huile de l'évaporateur à 80°C et distille l'oxazole sous une pression de 40 mm. Après distillation complète de l'oxazole on séche le support imprégné puis lui fait subir un traitement thermique analogue à celui décrit dans l'exemple 7. L'analyse indique une teneur en argent de 15,8 % en poids dans le catalyseur. On charge 30 ml de celui-ci dans le réacteur décrit dans l'exemple 10 et après un traitement d'activation effectué à pression atmosphérique sous un courant air-éthylène à 50 %—50 % pendant 48 heures entre 150° et 168°C, on fait passer sous 20 bars un débit de 9 000 litres normaux par heure et par litre de catalyseur d'un mélange gazeux contenant 13 % d'éthylène, 5 % d'oxygène, 82 % d'azote et 35 ppb de dichloro-1,2 éthane. A 190°C

pour un taux de transformation globale de l'éthylène de 4 %, la sélectivité en oxyde d'éthylène est de 70 %.

Exemple 12

Dans un ballon à solides monté sur un évaporateur rotatif, on charge 42,5 g de support NORTON SA 5239 de granulométrie 1/4 de pouce. On dégaze le support dans des conditions identiques à celles décrites dans l'exemple 7. On introduit goutte à goutte sur ce support une solution préalablement filtrée de 16,5 g d'acétate d'argent dans 112 g d'isoxazole et 27,1 g d'eau de façon que l'isoxazole et l'eau distillent au fur et à mesure de leur introduction. Après avoir totalement additionné la solution on sèche le support imprégné et lui fait subir un traitement thermique dans les conditions décrites dans l'exemple 7. Le dosage indique une teneur en argent de 12,7 % en poids dans le catalyseur. On place 30 ml de celui-ci dans le réacteur décrit dans l'exemple 10 et après un traitement d'activation effectué à pression atmosphérique sous courant air-éthylène à 50 %—50 % pendant 48 heures entre 150° et 195°C, on fait passer sous 20 bars, un débit de 9 000 litres normaux par heure et par litre de catalyseur d'un mélange gazeaux contenant 13 % d'éthylène, 5 % d'oxygène, 82 % d'azote et 35 ppb de dichloro-1,2 éthane. A 217°C pour un taux de transformation globale de l'éthylène de 5 %, la sélectivité en oxyde d'éthylène est de 75 %.

**Revendications**

1. Catalyseurs de synthèse de l'oxyde d'éthylène par oxydation en phase vapeur de l'éthylène par l'oxygène ou par un mélange gazeaux en contenant, préparés par imprégnation de supports réfractaires à l'aide de composées organo-métalliques de l'argent et traitement thermique du produit obtenu de manière à libérer l'argent métalliques, caractérisés par l'emploi comme composés organo-métalliques de complexes de sels d'argent avec des hétérocycles contenant trois atomes de carbone, un atome d'oxygène et un atome d'azote, choisis dans le group formé par l'oxazole, l'oxazoline-2, l'oxazoline-3, l'isooxazole, l'isooxazoline-2 et leurs dérivé substitués sur le carbone par un ou plusieurs groupements alkyle ayant de 1 à 10 atomes de carbone et/ou par un ou plusieurs groupements phényle.

2. Catalyseurs selon la revendication 1, où l'hétérocycle utilisé comme agent complexant est l'oxazole.

3. Catalyseurs selon la revendication 1, où l'hétérocycle utilisé comme agent complexant est la méthyl-2-oxazoline-2.

4. Catalyseurs selon l'une des revendications 1 à 3, caractérisés en ce que le complexe de sel d'argent est utilisé en solution dans un excès d'agent complexant.

5. Catalyseurs selon l'une des revendications 1 à 3, caractérisés en ce que le complexe de sel d'argent est utilisé en solution dans des solvants comme l'eau, les solutions aqueuses ammoniacales, les nitriles, les alcools, les cétones, les glycols légers et les amines.

6. Catalyseurs selon la revendication 5, caractérisés en ce que la solubilisation du complexe de sel d'argent dans les solvants est favorisée par l'utilisation d'au moins deux moles d'agent complexant par mole d'argent.

7. Catalyseurs selon l'une des revendications 4 à 6, caractérisés en ce que le sel d'argent est formé in situ par réaction de l'oxyde d'argent avec l'acide correspondant au sel, utilisé en quantité comprise entre 100 et 125 % de la stoechiométrie.

8. Catalyseurs selon l'une des revendications 1 à 7, caractérisés en ce que le sel d'argent utilisé est un sel d'un acide mono- ou di-carboxylique, contenant de 1 à 10 atomes de carbone.

9. Catalyseurs selon la revendication 8, caractérisés en ce que le sel d'argent utilisé est l'acétate d'argent.

10. Catalyseurs selon l'une des revendications 1 à 9, caractérisés en ce qu'ils contiennent de 5 à 25 % d'argent métallique.

11. Catalyseurs selon l'une des revendications 1 à 10, caractérisés par l'utilisation comme support réfractaire de supports alumineux et silico-alumineux de surface spécifique inférieure à 1 $m^2/g$ et de porosité apparente d'au moins 40% en volume, principalement formée de pores de rayons supérieurs à 0,25 micromètre.

12. Procédé de préparation d'oxyde d'éthylène par oxydation de l'éthylène en phase vapeur par l'oxygène ou par un mélange gazeux en contenant, caractérisé par l'emploi d'un catalyseur selon l'une des revendications 1 à 11.

**Claims**

1. Catalysts for the synthesis of ethylene oxide by oxidation of ethylene, in the vapour phase by means of oxygen or a gaseous mixture containing oxygen, said catalysts being prepared by the impregnation of refractory supports with organometallic silver compounds and thermal treatment of the product obtained so as to free the metallic silver, characterised by the use, as organometallic compounds of complexes of silver salts with heterocyclic groups containing three carbon atoms, one oxygen atom and

one nitrogen atom, selected from the group consisting of oxazole, 2-oxazoline, 3-oxazoline, isoxazole, 2-isoxazoline and the derivatives thereof substituted at the carbon by one or more alkyl groups having from 1 to 10 carbon atoms and/or by one or more phenyl groups.

2. Catalysts according to claim 1, wherein the heterocyclic group used as the complexing agent is oxazole.

3. Catalysts according to claim 1, wherein the heterocyclic group used as the complexing agent is 2-methyl-2-oxazoline.

4. Catalysts according to one of claims 1 to 3, characterised in that the silver salt complex is used in solution in an excess of complexing agent.

5. Catalysts according to one of claims 1 to 3, characterised in that the silver salt complex is used in solution in solvents such as water, aqueous ammoniacal solutions, nitriles, alcohols, ketones, light glycols and amines.

6. Catalysts according to claim 5, characterised in that the solubilising of the silver salt complex in the solvents is promoted by the use of at least two moles of complexing agent per mole of silver.

7. Catalysts according to one of claims 4 to 6, characterised in that the silver salt is formed *in situ* by reacting silver oxide with the acid corresponding to the salt, used in an amount of between 100 and 125% of stoichiometry.

8. Catalysts according to one of claims 1 to 7, characterised in that the silver salt used is a salt of a mono- or dicarboxylic acid, containing from 1 to 10 carbon atoms.

9. Catalysts according to claim 8, characterised in that the silver salt used is silver acetate.

10. Catalysts according to one of claims 1 to 9, characterised in that they contain from 5 to 25 % of metallic silver.

11. Catalysts according to one of claims 1 to 10, characterised by the use, as refractory supports, of aluminous and silico-aluminous supports with a specific surface of less than 1 $m^2/g$ and an apparent porosity of at least 40 % by volume, principally formed by pores with radii greater than 0.25 microns.

12. Process for preparing ethylene oxide by the oxidation of ethylene in the vapour phase by means of oxygen or a gaseous mixture containing oxygen, characterised by the use of a catalyst according to one of claims 1 to 11.

**Patentansprüche**

1. Katalysatoren zur Synthese von Äthylenoxid durch Dampfphasenoxydation von Äthylen mit Sauerstoff oder einer diese enthaltenden Gasmischung, hergestellt durch Imprägnierung von feuerfesten Trägern mit Hilfe von metallorganischen Silberverbindungen und thermische Behandlung des erhaltenen Produktes um metallisches Silber freizusetzen, dadurch gekennzeichnet, daß als metallorganische Verbindungen Komplexe von Silbersalzen mit drei Kohlenstoffatome, ein Sauerstoffatom, un ein Stickstoffatom umfassenden Heterocyclen verwendet werden, die aus der durch Oxazol, 2-Oxazolin, 3-Oxazolin, Isooxazol, 2-Isooxazolin und deren am Kohlenstoff durch eine oder mehrere Alkylgruppen mit 1 bis 10 Kohlenstoffatomen und oder einer oder mehreren Phenylgruppen substituierten Derivate gebildeten Gruppe ausgewählt sind.

2. Katalysatoren nach Anspruch 1, bei denen der als Komplexbildner verwendete Heterozyklus Oxazol ist.

3. Katalysatoren nach Anspruch 1, bei denen der als Komplexbildner verwendete Heterozyklus 2-Methyl-2-oxazolin ist.

4. Katalysatoren nach einem der Ansprüche 1 bis 3, dadurch gekennzeichnet, daß der Silbersalzkomplex in Lösung in einem Überschuß des Komplexbildners verwendet wird.

5. Katalysatoren nach einem der Ansprüche 1 bis 3, dadurch gekennzeichnet, daß der Silbersalzkomplex in Lösung in Lösungsmitteln wie Wasser, wässrigen Ammoniaklösungen, Nitrilen, Alkoholen, Ketonen, niederen Glykolen und Aminen verwendet wird.

6. Katalysatoren nach Anspruch 5, dadurch gekennzeichnet, daß die Löslichkeit des Silbersalzkomplexes in den Lösungsmitteln durch Verwendung von mindestens zwei Molen des Komplexbildners pro Mol Silber begünstigt ist.

7. Katalysatoren nach einem der Ansprüche 4 bis 6, dadurch gekennzeichnet, daß das Silbersalz in situ durch Mischung von Silberoxid mit der dem Salz entsprechenden Säure gebildet ist, die in einer Menge zwischen 100 und 125% der stöchiometrischen Menge verwendet wird.

8. Katalysatoren nach einem der Ansprüche 1 bis 7, dadurch gekennzeichnet, daß das verwendete Silbersalz ein Salz einer Mono- oder Dicarbonsäure mit 1 bis 10 Kohlenstoffatomen ist.

9. Katlysatoren nach Anspruch 8, dadurch gekennzeichnet, daß das verwendete Silbersalz Silberacetat ist.

10. Katalysatoren nach einem der Ansprüche 1 bis 9, dadurch gekennzeichnet, daß sie 5 bis 25 Prozent metallisches Silber enthalten.

11. Katalysatoren nach einem der Ansprüche 1 bis 10, gekennzeichnet durch die Verwendung von aluminatischen und silico-aluminatischen Trägern mit einer spezifischen Oberfläche unter 1 $m^2/g$ und einer scheinbaren Porosität von mindestens 40 Volumenprozent, die hauptsächlich durch Poren mit Durchmessern im Bereich über 0,25 $\mu m$ bedingt ist, als feuerfeste Träger.

12. Verfahren zur Herstellung von Äthylenoxid durch Dampfphasenoxydation von Äthylen mit Sauerstoff oder einer diesen enthaltenen Gasmischung, gekennzeichnet durch die Verwendung eines Katalysators nach einem der Ansprüche 1 bis 11.